Europäisches Patentamt

European Patent Office

Office européen des brevets

(11) Publication number: **0 320 170**
**A1**

## EUROPEAN PATENT APPLICATION

(21) Application number: **88311374.8**

(22) Date of filing: **01.12.88**

(51) Int. Cl.4: **A61F 2/78**

(30) Priority: **11.12.87 GB 8729032**

(43) Date of publication of application:
**14.06.89 Bulletin 89/24**

(84) Designated Contracting States:
**AT BE CH DE ES FR GB GR IT LI LU NL SE**

(71) Applicant: **W.L. GORE & ASSOCIATES, INC.**
**555 Paper Mill Road P.O. Box 9206**
**Newark Delaware 19714(US)**

(72) Inventor: **Norman, Edward George**
**Forthview Hawkcraig Point**
**Aberdour Fife Scotland KY3 0TZ(GB)**

(74) Representative: **Taylor, Derek George et al**
**MATHISEN, MACARA & CO. The Coach**
**House 6-8 Swakeleys Road**
**Ickenham Uxbridge UB10 8BZ(GB)**

(54) A protective cover for an amputated limb stump.

(57) A protective cover (10) for an amputated limb stump is formed from microporous expanded PTFE membrane, by stretching the membrane over a former shaped to correspond to the limb stump, and locking the shape of the membrane by the application of heat. The membrane can be permanently secured to a supporting rim (10a), or to an absorbent sock (13) for which it forms a liner.

FIG.2.

EP 0 320 170 A1

## A PROTECTIVE COVER FOR AN AMPUTATED LIMB STUMP

This invention relates to a protective cover for an amputated limb stump.

Covers currently used for this purpose are 100% wool socks which are intended to protect the stump from the very high pressures exerted on the boney end when an artificial limb prothesis is fitted to the stump. This applies particularly to a leg stump.

Amputees suffer greatly from the profuse perspiration of a stump, particularly a leg stump. The fluid which builds up in the covering sock causes the skin covering of the stump to become white and to deteriorate leading to breakdown of skin if not treated. Certainly severe discomfort is caused, and the sock forms a breeding ground for bacteria, and may set up infection.

These conditions are considerably relieved by means of the present invention.

According to the present invention there is provided a protective cover for an amputated limb stump comprising a cup-shaped membrane of microporous expanded polytetrafluoroethylene (PTFE) which is hydrophobic but capable of transmitting water vapour.

Preferably the cover is shaped by stretching a sheet of microporous expanded PTFE on to a former and locking the cover in its stretched shape by the application of heat. The PTFE membrane and its method of manufacture is described in GB-A-1,355,373 and is sold by W. L. Gore & Associates Limited under the trade mark "GORE-TEX". The term 'hydrophobic' signifies the membrane's resistance to the transmission of aqueous media in the liquid state, whilst the term 'water vapour permeable' signifies the ability of the membrane to transmit water vapour and gases through the pore structure.

The present invention also provides a method of forming a protective cover for an amputated limb stump which comprises stretching a sheet of microporous expanded PTFE on to a former shaped to correspond with a limb stump and locking the cover in its stretched state by the application of heat.

An embodiment of the present invention is illustrated in the accompanying drawings in which:-

Figure 1 is a diagrammatic representation of a process for shaping a limb stump cover in accordance with the invention,

Figure 2 is an exploded diagrammatic view of a cover being fitted to a limb stump, and

Figure 3 is a diagrammatic view of a limb stump and cover to which an artificial limb has been fitted.

Figures 4 and 5 are diagrammatic views of an alternative way of fitting a limb stump cover to a limb stump.

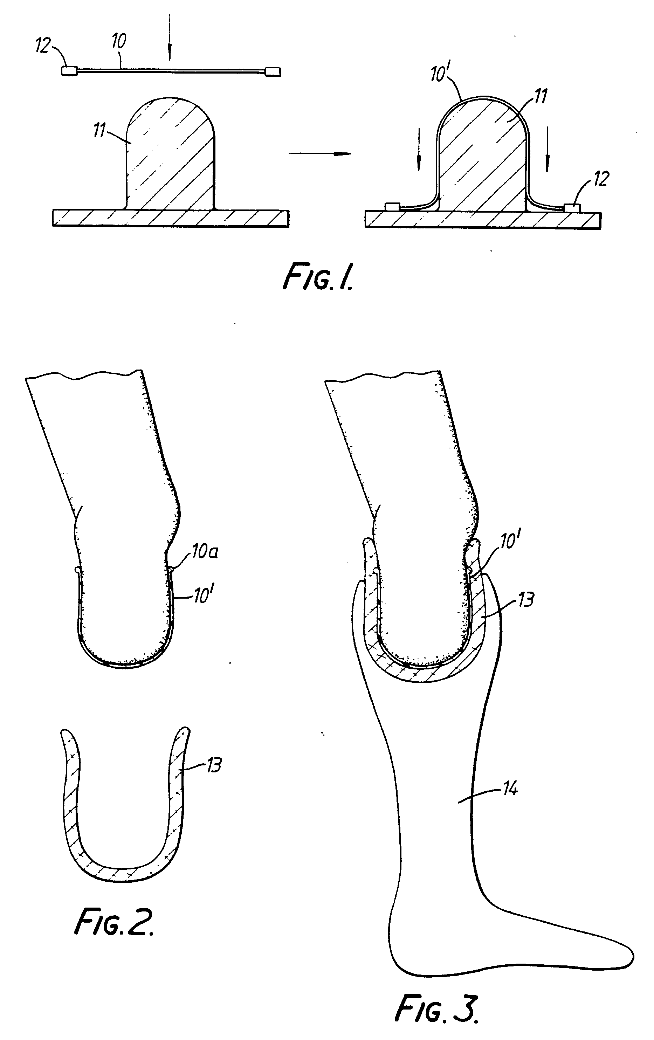

As illustrated in Figure 1, a limb stump cover can be made from a sheet or membrane 10 of microporous expanded hydrophobic P.T.E. by stretching the sheet over a former 11 profiled to the shape of the limb stump to be covered. Conformation of the membrane to the former may be enhanced by applying vacuum suction around the base of the former. Preferably the membrane is in a non-heat-locked state while it is stretched, but it is then locked in the shape by the application of heat, to form the profiled cover 12. For example, the former may be preheated to between 50° and 100°C or the membrane cover may be heat-set and locked by brief exposure to temperatures between 350° and 400°. This brief exposure could be in an oven, or by air blast or infra-red radiation.

To assist in the stretch forming process, the membrane may be held in a circular clamp frame which is then passed downwardly over the former.

The membrane is suitably of 0.0035" (0.0089cm) thickness and can be supported by a rim 10a fitted thereto before or after stretching. This rim support could be provided by reinforcing the membrane with elasticated fabric or elastic cord, and such support could improve retention of the cover on the limb stump.

The outer rim of the pre-formed membrane can be sewn or otherwise attached to the rim of an absorbent outer sock 13. Alternatively the membrane can be pre-laminated to a suitable thermoplastic (such as knitted nylon, acrylic or polyester fibres) and subsequently pressure formed to the desired profile shape using matched former moulds and forming temperatures between 200°C and 250°C.

Figures 2 and 3 show the shaped cover 10′ fitted to a limb stump and covered with an absorbent outer sock l3 to assist in providing a cushion between the limb stump and the artificial limb l4. The sock in this event absorbs the perspiration which has passed as water vapour through the membrane cover 12, and enables it to evaporate from exposed surfaces of the sock. The perspiration which collects as liquid in the sock however, is prevented from passing back through the membrane to the limb stump in liquid form by virtue of the hydrophobic characteristic of the membrane.

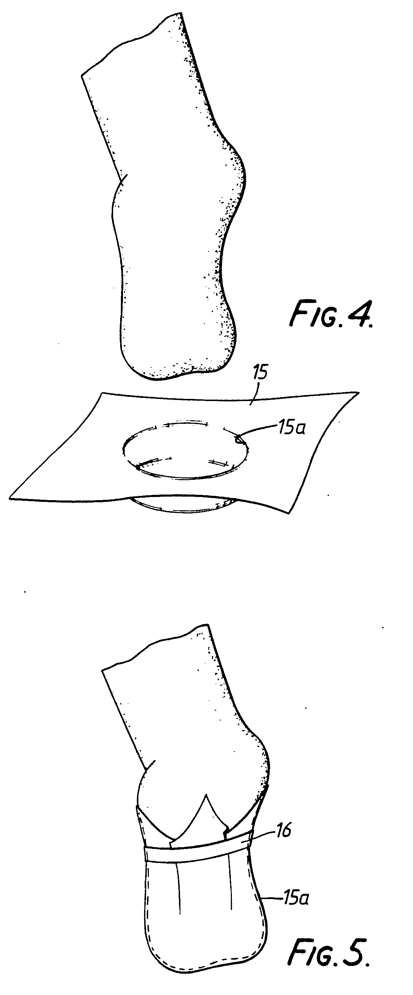

Figure 4 shows a rectangular sheet 15 of microporous expanded P.T.F.E. which has been cold formed to provide a cup shape 15a in the centre. Figure 5 shows the cup-shaped sheet having been fitted over a limb stump, the edges of the sheet folded against the limb stump and secured by a band 16 of adhesive surgical tape wrapped around the folded sheet prior to covering with an absorbent wool or acrylic sock.

## Claims

1. A protective cover for an amputated limb stump comprising a cup-shaped membrane of microporous expanded polytetrafluoroethylene (PTFE) which is hydrophobic but capable of transmitting water vapour.

2. A cover according to claim 1 which has been shaped by stretching a sheet of microporous expanded PTFE on to a former and locking the cover in its stretched shape by the application of heat.

3. A cover according to claim 1 or claim 2 of which a rim is permanently secured to a knitted fabric layer, and forms a lining thereof.

4. A cover according to any preceding claim of which the outer rim is reinforced with elasticated fabric or elastic cord.

5. A method of forming a protective cover for an amputated limb stump which comprises stretching a sheet of microporous expanded PTFE on to a former shaped to correspond with a limb stump and locking the cover in its stretched state by the application of heat.

6. A method according to claim 5 wherein the cover is exposed briefly to temperatures between 350° and 400°.

*FIG. I.*

*FIG. 2.*

*FIG. 3.*

EP 0 320 170 A1

*FIG.4.*

15

15a

*FIG.5.*

16

15a

## EUROPEAN SEARCH REPORT

European Patent Office

# DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-3 600 717 (McKEEHAN)<br>* Column 2, lines 20-22; abstract; figures * | 1,2,5,6 | A 61 F 2/78 |
| D,Y | GB-A-1 355 373 (GORE & ASSOCIATES)<br>* Whole document * | 1,2,5,6 | |
| A | US-A-4 635 626 (LERMAN) | | |
| A | GB-A- 383 229 (DAVIES) | | |
| A | DE-A-2 917 478 (BIEHL) | | |

TECHNICAL FIELDS SEARCHED (Int. Cl.4)

A 61 F

The present search report has been drawn up for all claims

| Place of search | Date of completion of the search | Examiner |
|---|---|---|
| THE HAGUE | 16-03-1989 | STEENBAKKER J. |

EPO FORM 1503 03.82 (P0401)